# EUROPEAN PATENT SPECIFICATION

(11) **EP 1 562 629 B1**
(45) Date of publication and mention of the grant of the patent: **03.06.2009**
(21) Application number: 03770160.4
(22) Date of filing: 03.10.2003
(51) Int. Cl.: A61K 38/17, A61P 9/10

(54) **THERAPEUTIC USES OF BETA-CASEIN A2 AND DIETARY SUPPLEMENT CONTAINING BETA-CASEIN A2**
THERAPEUTISCHE ANWENDUNGEN VON BETA-CASEIN A2 UND NAHRUNGSERGÄNZUNG MIT BETA-CASEIN A2
UTILISATIONS THERAPEUTIQUES DE LA BETA-CASEINE A2 ET D'UN COMPLEMENT ALIMENTAIRE CONTENANT DE LA BETA-CASEINE A2

(30) Priority: 04.10.2002 NZ 52195502
(43) Date of publication of application: 17.08.2005
(73) Proprietor: A2 Corporation Limited, Newmarket, Auckland (NZ)
(72) Inventor: CAMPBELL, Julie Hazel, Brookfield, QLD 4069 (AU); TAILFORD, Kristy Ann, Carseldine, Queensland 4034 (AU); MCLACHLAN, Ulrike HF, Devonport, Auckland (NZ); McLACHLAN, Corran Norman Stuart, deceased (NZ)
(74) Representative: Lees, Kate Jane
(86) International application number: PCT/NZ2003/000222
(87) International publication number: WO 2004/030690

(56) References cited:
- WO-A-01/00047
- WO-A-96/36239
- JP-A- 6 165 655
- DEMLING R H ET AL: "Effect of a hypocaloric diet, increased protein intake and resistance training on lean mass gains and fat mass loss in overweight police officers" ANNALS OF NUTRITION AND METABOLISM, KARGER, CH, vol. 44, no. 1, 2000, pages 21-29, XP009087034 ISSN: 0373-0101
- BEALES P. E. ET AL.: 'A multi-centr, blinded international trial of the effect of A1 and A2 B-casein variants on diabetes incidence in two rodent models of spontaneous Type I diabetes' DIABETOLOGIA vol. 45, no. 9, September 2002, pages 1240 - 1246, XP009086999
- TAILFORD KRISTY A. ET AL.: 'A casein variant in cow's milk is atherogenic' ATHEROSCLEROSIS vol. 170, no. 1, September 2003, pages 13 - 19, XP003016352
- MCLACHLAN C. N. S.: 'B-casein A1, ischemic heart disease mortality, and other illnesses' MEDICAL HYPOTHESES vol. 56, no. 2, February 2001, pages 262 - 272, XP005701627
- JIM MANN AND MURRAY SKEAFF: 'Beta-casein variants and atherosclerosis -claims are premature' ATHEROSCLEROSIS vol. 170, no. 1, September 2003, pages 11 - 12, XP003016353

## Description

### TECHNICAL FIELD

This invention relates to the use of β-casein A² for the reduction of certain disease risk factors, such as cholesterol, lipid, and triglyceride levels, and LDL:HDL ratios. In particular, the invention relates to the prevention or treatment of diseases or disorders associated with elevated levels of such risk factors. The invention further relates to a dietary supplement containing β-casein A².

### BACKGROUND

Cardiovascular disease is widespread and prevalent in the western world today. The search for the prevention and treatment of this disease, and for the risk factors associated with it, is an ongoing challenge.

Hypercholesterolemia is implicated as a high risk factor for cardiovascular diseases including arteriosclerosis, atherosclerosis and xanthomatosis. Hypercholesterolemia is influenced by diet, heredity, environment, life style, other diseases and stress, leading to heart attacks and strokes at an early age.

Hypercholesterolemia is a condition with elevated levels of circulating total cholesterol, (low density lipoprotein) LDL-cholesterol and (very low density lipoprotein) VLDL-cholesterol. In particular, high levels of LDL and VLDL are positively associated with coronary arteriosclerosis while high levels of HDL are negative risk factors. The role of LDL oxidation has gained much attention in the literature. It is well documented that modified LDL, or more specifically oxidised LDL, has an increased ability to bind endothelial and smooth muscle cell walls thus promoting further injury by continued oxidation within the lumen of arteries.

Furthermore, oxidised LDL is known to invoke a range of physiological responses directly involved in the atherosclerotic process. These responses include the stimulation of the secretion of mediators of inflammatory response (e.g. interleukin-1, interleukin-6, tumour necrosis factor alpha and macrophage colony stimulating factor) which are chemo-attractants for macrophages which recognise the oxidised LDL within the subendothelial space and actively engulf them, subsequently turning into foam cells. The foam cells then aggregate to form fatty streaks. Such fatty streaks are the first identifiable characteristic lesion of advanced atherosclerosis.

Hyperlipidemia also predisposes one to coronary heart disease, as well as to cancer and obesity. Hyperlipidemia is one of the high risk factors useful in the early diagnosis of these life threatening diseases.

Hyperlipidemia is a condition where the blood lipid parameters are elevated. The lipids fractions in the circulating blood are total cholesterol, LDL, VLDL, and triglycerides. Safe levels of these according to the American Heart Association guidelines are represented below. Active treatment by diet modifications and drugs are necessary to reduce the risk of fatality when the levels are abnormal.

| | |
|---|---|
| Total Cholesterol | (TC) >240 mg/dL |
| LDL-Cholesterol | >160 mg/dL - Apo(B) Atherogenic factor |
| HDL-Cholesterol | <35 mg/dL Lp(a) Atherogenic factor |
| Triglycerides | >150 mg/dL |

As with hypercholesterolemia, hyperlipidemia results from diet, heredity, lifestyle, environment, familial diseases, or stress. The condition may be inherited or may be secondary to another disorder, such as systemic lupus erythematosus (SLE), hypothyroidism, nephrotic syndrome, Cushing's syndrome, diabetes mellitus, obesity, alcoholism, corticosteroid therapy or estrogen therapy.

Hypercholesterolemia and hyperlipidemia are also major causes of atherosclerosis. Atherosclerosis is responsible for more deaths in the U.S. than any other single condition. Atherosclerotic heart disease involving the coronary arteries is the most common single cause of death, accounting for one third of all deaths. Atherosclerotic interference with blood supply to the brain (causing stroke) is the third most common cause of death (after cancer). Atherosclerosis also causes a great deal of serious illness by reducing the blood flow in other major arteries, such as those to the kidneys, the legs and the intestines.

Atherosclerosis is a cardiovascular condition that occurs as a result of narrowing down, or stenosis, of the arterial walls. The narrowing is due to the formation of plaques (raised patches) or streaks in the inner lining of the arteries. These plaques consist of oxidised-LDL, monocytes, macrophages, foam cells, damaged muscle cells, fibrous tissue, clumps of blood platelets, cholesterol, and sometimes calcium. They tend to form in regions of turbulent blood flow and are found most often in people with high concentrations of cholesterol in the bloodstream. The number and thickness of plaques increases with age, causing loss of the smooth lining of the blood vessels and encouraging the formation of thrombi (blood clots). It is not uncommon for fragments of thrombi to break off and form emboli, which travel through the bloodstream and block smaller vessels (e.g. coronary arteries) leading to ischaemic damage of tissue, or ischaemic heart disease.

Medication is not a satisfactory treatment for ischaemic heart disease because much of the damage to the artery walls has already been done. Anticoagulant drugs have been used to try to minimise secondary clotting and embolus formation, but these have little or no effect on the progress of the disease. Vasodilator drugs are used to provide symptom relief, but are of no curative value.

Surgical treatment is available for certain high-risk situations. Balloon angioplasty can open up narrowed vessels and promote an unproved blood supply. The blood supply to the heart muscle, following coronary vessel blockage or damage, can also be restored through a vein graft bypass. Large atheromas and calcified arterial obstructions can be removed by endarterectomy, and entire segments of diseased peripheral vessels can be replaced by woven plastic tube grafts.

In some instances the reduction of hypercholesterolemia can be achieved by modification of the diet and/or use of drugs thereby minimising the risk of fatality from the disease. Reduction of serum cholesterol in humans has been achieved by consumption of dietary plant fibre and other effective components of foods. However, there remains a need for a safe and effective treatment for the above conditions.

In addition to the other risk factors noted above, there is now considerable evidence linking consumption of milk, particularly the milk of *Bos taurus* cows (domestic cows), with diseases such as diabetes, atherosclerosis and coronary heart disease. The effect on hypercholesterolemia was shown in JP 6165655 A disclosing use of casein whey and other whey proteins.

The casein composition of milk obtained from bovine herds around the world has therefore been investigated in some detail, with particular reference to causative links between different caseins and diseases such as diabetes and coronary heart disease. Attention has focused on the β-casein, found in bovine milk.

Eight variants of the β-casein protein are known. These are identified as β-caseins A¹, A², A³, B, C, D, E and F. The prevalent variants in the milk of the world's bovine populations are β-casein A¹ and β-casein A². The distinction between them at the level of primary structure, or amino acid sequence, is that β-casein A¹ has the amino acid histidine at position 67 whereas β-casein A² has the amino acid proline at position 67. The other β-casein variants, though they may differ at other amino acid positions, can also be distinguished on the basis of whether histidine or proline resides at position 67. Thus, β-caseins B, C and F have histidine at position 67 and β-casein A³, D and E have proline at position 67.

Epidemiological evidence strongly suggests that dietary β-casein A¹ is harmful to human health. For example, WO 96/14577 describes the impact of β-casein A¹ on Type I diabetes. The epidemiological evidence suggests that β-casein A¹ stimulates diabetogenic activity in humans. Furthermore, WO 96/14577 describes the induction of autoimmune, or Type I, diabetes in the non-obese diabetic (NOD) mouse model by way of consumption of β-casein A¹. The invention described focuses on reducing the risk of contracting Type I diabetes in a susceptible individual by restricting the milk or milk product intake of that individual to milk containing only non-diabetogenic β-casein variants.

WO 96/36239 advocates the avoidance of β-casein A¹ in the human diet. Epidemiological evidence establishes a correlation between the consumption of β-casein A¹ in various populations and the incidence of coronary heart disease. The invention described in that document focuses on the avoidance of β-casein A¹ by selecting milking cows by testing genetic material for DNA encoding the various β-caseins.

Thus, the link between β-casein A¹ and diabetes and the link between β-casein A¹ and coronary heart disease have both been well documented. Additionally, it has recently been shown that the deleterious effects of β-casein A¹ now extend to neurological disorders. WO 02/19832 describes the avoidance of inducing or aggravating a neurological or mental disorder by providing milk which does not contain any "histidine variant". A histidine variant is defined in that document as a β-casein variant which has histidine at position 67. β-Casein A¹ is such a histidine variant.

The current literature focuses on the finding that β-casein A¹ is harmful to health, and therefore that β-casein A¹ should be eliminated from the diet of human populations. Most herds of cows produce milk which contains predominantly β-casein A¹ and β-casein A², and little (if any) of the other β-casein variants. The desire to eliminate β-casein A¹ from the dietary intake of a population can therefore be achieved by providing milk for human consumption which contains only β-casein A² of the possible β-casein variants. In other words, the drive for milk (and foods processed from that milk) to contain only β-casein A² has been motivated by the avoidance of β-casein A¹ and the consequent minimising of risks associated with diabetes, coronary heart disease, and neurological disorders.

WO 01/00047 discloses a dietary supplement comprising a milk product which contains predominantly the A² variant of the β-caseins, and which is fortified with a compound (or compounds) that lowers human plasma homocyst(e)ine levels. Such compounds may include betaine, cobalamin, folic acid or pyridoxine. Two possible advantages to this are described. Firstly, the replacement of β-casein A¹ in the supplement with β-casein A² will lower the risk of diabetes. Secondly, it is desirable to lower homocyst(e)ine levels, since these are highly correlated with coronary heart disease, and homocyst(e)ine is also a recognised risk factor for atherosclerosis. Thus, there is a combined approach, using the homocyst(e)ine strategy together with the diabetes strategy, for controlling vascular disease.

WO 01/00047 further discloses that β-casomorphin-9, the putative digestion product of β-casein A², may provide immunity against diabetes. Experimental results showed that rats fed with β-casein A² had a lower incidence of diabetes than control animals. β-Casomorphin-9 is described as a modulator of immune function, and therefore a possible attenuator of the diabetes disease process.

However, it should be noted that the data pertaining to immune modulation by β-casomorphin-9 were based on experiments that used injected, rather than gut-accessed, antigens on the NOD mouse model of diabetes. Furthermore, it is still not established that β-casomorphin-9 is the single active compound produced by digestion of β-casein A². Thus, it is difficult to extrapolate data based on β-casomorphin-9 and accurately predict whether β-casein A² will have the same biological activity.

It is noted in WO 01/00047 that there is a causal connection between diabetes and coronary heart disease. However, the suggestion that the diabetes-attenuating properties of β-casomorphin-9 mean β-casein A² may, at least indirectly, have a protective effect against coronary heart disease is based on an entirely tenous link and is considered a surprising suggestion given the experiments conducted.

The inventors have now, for the first time, surprisingly found that consumption of β-casein A² reduces serum cholesterol levels, reduces circulating triglyceride levels, reduces LDL concentrations and thus LDL:HDL ratios, reduces serum levels of apolipoprotein B and is atheroprotective.

It is therefore an object of the invention to provide a medical use of preventing or treating various diseases associated with mammalian serum cholesterol levels, LDL cholesterol levels or triglyceride levels by the ingestion of a composition containing β-casein A² in sufficient amounts to have a preventative or therapeutic effect against such diseases, or to at least provide a useful alternative.

### STATEMENTS OF INVENTION

According to the invention there is provided a β-casein for use in the treatment of hypercholesterolemia, hyperlipidemia and atherosclerosis by reducing the serum level in a mammal of any one or more of the following:
a) cholesterol;
b) low density lipoprotein (LDL) cholesterol relative to high density lipoprotein (HDL) cholesterol;
c) low density lipoprotein (LDL) cholesterol;
d) very low density lipoprotein (VLDL) cholesterol;
e) apolipoprotein B; and
f) triglycerides
where the β-casein is comprised of at least 95% β-casein A². Preferably, the β-casein is comprised solely of β-casein A².

It is preferred that the composition is a food or food product, such as milk or a product containing or processed from milk.

In a preferred embodiment of the invention, the composition is a dietary supplement, preferably a nutriceutical which is ingested for optimizing any one or more of the group consisting of performance during exercise, weight loss, weight gain, muscle building, and muscle repair.

It is further preferred that the composition has been formed by the addition of an amount of β-casein A² sufficient to alter serum cholesterol levels and/or serum triglyceride levels in a human.

Preferably the β-casein A² is obtained from the milk of bovine cattle or from the milk of yaks, buffalo, sheep or goats.

### BRIEF DESCRIPTION OF FIGURES

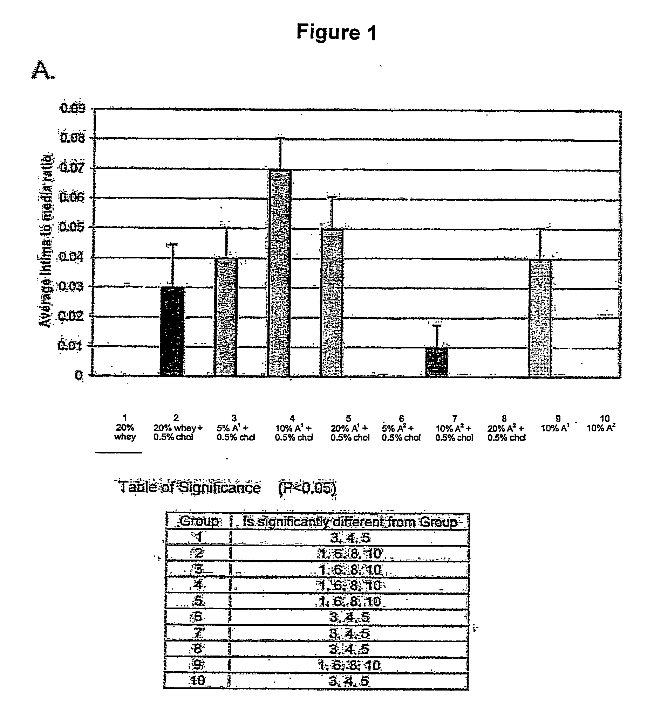

**Figure 1** shows average intima to media ratios (indicative of arterial thickening) of the aorta, for β-casein A², β-casein A¹ and whey-fed NZ white rabbits.
**Figure 2** shows the extent of neointimal thickening of the de-endothelialised right carotid artery (indicative of arterial disease progression following insult to arterial walls). A: Example from Group 1 showing no neointimal thickening, B: Example from Group 2 showing some lesion formation in the intimal layer, and C: Example from Group 5 showing severe intimal thickening.

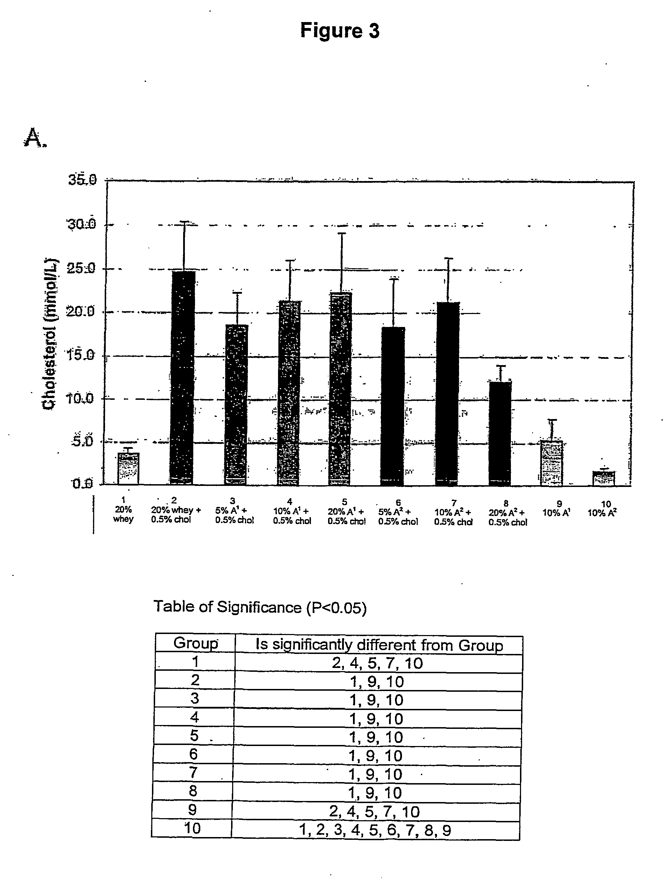

**Figure 3** shows serum cholesterol levels for β-casein A²-fed, β-casein A¹-fed and whey-fed NZ white rabbits.

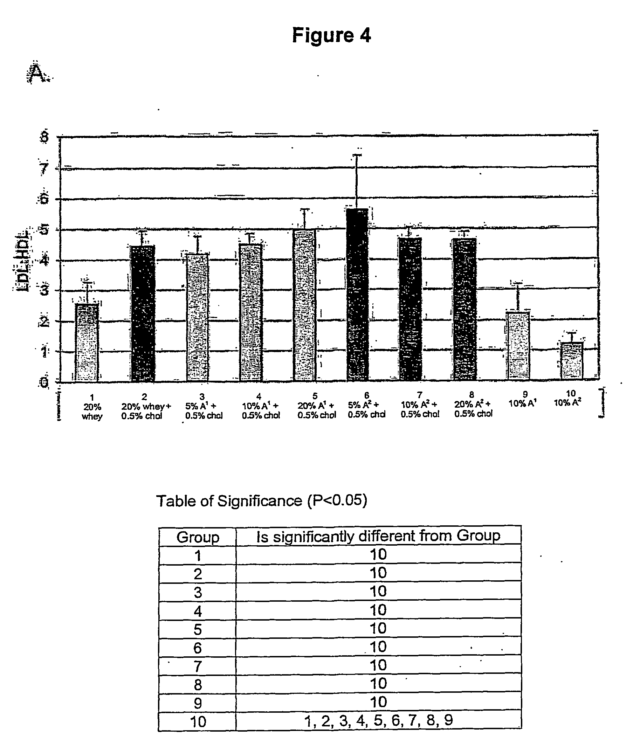

**Figure 4** shows serum LDL to HDL ratios for β-casein A²-fed, β-casein A¹-fed and whey-fed NZ white rabbits.

### DETAILED DESCRIPTION

As used herein the term "LDL" means low density lipoprotein, "HDL" means high density lipoprotein, and "VLDL" means very low density lipoprotein.

As used herein the term "triglyceride" means a lipid or neutral fat consisting of glycerol combined with three fatty acid molecules.

As used herein the term "cardiovascular disease" is any disease of the blood vessels of the circulation system caused by, facilitated by or shown to be linked to abnormally high concentrations of lipids in the vessels.

As used herein the term "arteriosclerosis" is a degeneration of the walls of the arteries due to the formation of foam cells and aortic streaks which narrow the arteries. This limits blood circulation and predisposes an individual to thrombosis.

As used herein the term "atherosclerosis" is a disease of the arteries in which fatty plaques develop on the inner walls, with eventual obstruction of blood flow.

As used herein the term "apolipoprotein B" or "apoprotein B" or "Apo B" refers to the protein component of the LDL cholesterol transport proteins. Cholesterol synthesised de novo is transported from the liver and intestine to peripheral tissues in the form of lipoproteins. Most of the apolipoprotein B is secreted into the circulatory system as VLDL.

As used herein the term "hypercholesterolemia" means elevated levels of circulating total cholesterol, LDL-cholesterol and VLDL-cholesterol as per the guidelines of the Expert Panel Report of the National Cholesterol Educational Program (NCEP) of Detection, Evaluation of Treatment of High Cholesterol in Adults (Arch. Int. Med. (1988) 148, 36-39).

As used herein the term "hyperlipidemia" or "hyperlipemia" is a condition where the blood lipid parameters are elevated in the blood. This condition manifests an abnormally high concentration of fats. The lipids fractions in the circulating blood are, total cholesterol, LDLs, VLDLs, and triglycerides.

As used herein the term "lipoprotein", such as in VLDL, LDL and HDL, refers to a group of proteins found in the serum, plasma and lymph which are important for lipid transport. The chemical composition of each lipoprotein differs in that the HDL has a higher proportion of protein versus lipid, whereas the VLDL has a lower proportion of protein versus lipid.

As used herein the term "xanthomatosis" is a disease evidence by a yellowish swelling or plaques in the skin resulting from deposits of fat. The presence of xanthomas are usually accompanied by raised blood cholesterol levels.

As used herein the term "GRAS" means "generally regarded as safe" with respect to food additives.

The inventors have shown for the first time the interesting and surprising result that consumption of β-casein A² has positive beneficial effects, including:
1. The lowering of serum cholesterol levels,
2. Reduction of circulating triglyceride levels, and
3. Reduction of LDL concentrations and LDL:HDL ratios.

These effects observed by the inventors mean that the consumption of β-casein A² is beneficial for the prevention or treatment of a variety of diseases or disorders including:
1. hypercholesterolemia,
2. hyperlipidemia, and
3. atherosclerosis.

This important finding alters the current thinking that β-casein A² is beneficial simply because β-casein, A¹ contributes to certain diseases, such as diabetes and coronary heart disease. The inventors' finding is that β-casein, A² has a positive beneficial effect in its own right.

The finding is supported by experimental studies where ten groups of NZ white rabbits were fed *ad libitum* with controlled diets. Following sacrifice of the animals, serum cholesterol levels, LDL:HDL ratios and triglyceride levels were measured.

In rabbit Groups 3 - 8, three concentrations of β-casein A¹ and A² (5%, 10% and 20%) in the diet were tested, with whey protein added such that a total of 20% milk protein was present in each diet (i.e. 15%, 10%, 0% added whey protein respectively). Cholesterol (0.5%) was also added to these diets to ensure measurable lesions. As controls, Group 1 rabbits were fed 20% whey protein and Group 2 rabbits were fed 20% whey protein plus 0.5% cholesterol. Groups 9 and 10 were fed 10% β-casein A¹ (or A²) plus 10% whey only. The rabbits on the specially prepared diet ate only an average of 30.05 ± 0.97 grams per day.

Not surprisingly, almost all rabbits lost weight over the 6 week experimental period, with a 5.62% average weight loss. This did not affect their overall health and the rabbits were alert, responsive and showed no signs of distress. This is consistent with an earlier study which found that, with rabbits fed a semisynthetic diet containing casein, the feed intake and weight gain was reduced by half as the diet was not readily accepted, although the rabbits still appeared healthy. Rabbits placed on a high level of dietary casein (50%) failed to gain weight during the course of that study.

10% β-casein A² with no added cholesterol (Group 10) produced a significantly lower serum cholesterol level at t = 6 weeks than all other Groups including whey only (Group 1) and 10% β-casein A¹ (Group 9). The β-casein A¹ Group had serum cholesterol levels higher than the whey only Group. This highlights the necessity to examine the effect of the specific β-caseins rather than mixtures, and is the first time that the differential effects of β-casein variants on serum cholesterol levels have been described.

The mean serum cholesterol level in rabbits fed 20% β-casein A¹ alone (Group 9) was higher than those in Group 1 (whey only) animals. Thus, β-casein A¹ may be slightly more atherogenic than whey protein. Adding 0.5% cholesterol to all diets increased the serum cholesterol levels at least 3-fold, which was much greater than the effect of A¹ β-casein compared with whey alone. There was no significant difference between the serum cholesterol levels of rabbits fed β-casein A¹ or A² in the presence of added dietary cholesterol.

The level of serum LDL in Group 10 β-casein A²) was significantly lower than that in Groups 9 (β-casein A¹) or 1 (whey only), all with no dietary cholesterol. The level of LDL for Group 9 was higher than that for Group 1, but not significantly. In the presence of added dietary cholesterol, serum LDL was significantly elevated in all Groups (2 - 8) and these were not significantly different from each other. A similar pattern occurred for serum HDL except that Group 10 was not significantly lower than Group 1.

The anti-atherogenic potential of β-casein A² was further highlighted by the ratios of LDL to HDL. There was a much greater difference in serum LDL levels between Groups 9 (β-casein A¹) and 10 (β-casein A²) than in serum HDL levels. Thus the LDL to HDL ratios were significantly less for β-casein A² fed animals than for β-casein A¹ fed animals. Indeed, the LDL to HDL ratio of Group 10 was significantly lower than all other groups, including Group 1 (20% whey alone). There was no significant difference between all groups for triglycerides or homocysteine.

Thus, dietary β-casein A², in the absence of a cholesterol-enriched diet, leads to lower serum cholesterol levels (total, LDL and HDL) than both β-casein A¹ and whey.

It was found that both β-casein A¹ and A² led to fatty streaks compared with 20% whey, but β-casein A² produced less extensive lesions than β-casein A¹. Indeed, 10% β-casein A¹ without dietary cholesterol produced about the same amount of lesions as 10% β-casein A² with added dietary cholesterol. The thickness of fatty streaks in the aortic arch of rabbits fed β-casein A², either with or without dietary cholesterol, was zero or else very close to zero. In contrast, the thickness of aortic arch fatty streaks in rabbits fed β-casein. A¹ was significantly higher. Thus, β-casein A¹ is more atherogenic than both whey and the A² casein variant.

In the presence of 0.5% dietary cholesterol, only 20% β-casein A² produced a smaller surface area of aorta covered by fatty streaks than 20% whey. However, the thickness of the fatty streaks in the aortic arch was significantly smaller for both 20% and 5% β-casein A²-fed animals compared with whey (0.03 ± 0.015), with a low (but not significant) value for 10% β-casein A² fed animals (0.01 ± 0.008).

Balloon injury of arteries combined with a cholesterol-enriched diet is known to produce lesions in rabbits that closely resemble advanced human atheroma.

There was no significant difference in intima to media ratio of the balloon injured right carotid artery of rabbits fed β-casein A¹ or A² either with or without dietary cholesterol. However, groups fed β-casein A² (both with and without added cholesterol) had smaller neointimal thickenings than their β-casein A¹ fed counterparts. Animals fed β-casein A² generally had slightly thinner intimas than those fed whey (both with and without dietary cholesterol).

These results, combined with the fact that 10% A² with no added cholesterol produced a significantly lower serum cholesterol level than all other groups including whey only and 10% β-casein A¹, demonstrate for the first time that β-casein A² has an atheroprotective effect while β-casein A¹ is atherogenic.

These results are consistent with the epidemiological studies, which suggest a strong relationship between mortality from cardiovascular disease and consumption of β-casein A¹.

The composition of the invention may be in the form of β-casein A² admixed with one or more substances, and may be in the form of an aqueous solution or suspension or emulsion, or may be a solid such as a granular or powder form. Certain compositions of the invention may be in their naturally occurring form, especially milk or products derived or processed from that milk. Other compositions of the invention may be substances supplemented by the addition of β-casein A², which β-casein A² has been purified from or obtained as a fraction from milk or a product processed from that milk, or which β-casein A² is present in milk or a product processed from that milk.

It will be clear to those skilled in the art that the composition or food product of the invention may include, but is not limited to, milk or milk products, nutritional supplements, for example weight gain formulations, food additives, ice-cream or other frozen dairy based confections, fermented milk products such as yoghurt or quark, cheeses including full fat, partial de-fatted and fat-free processed cheeses, milk whey, food products enriched through the addition of milk products such as soups, milk from which allergenic molecules have been removed, confections such as chocolate, carbonated milk products, including those with added phosphate and/or citrate, infant formulations which may contain full, partially de-fatted or nonfat milk, liquid or powdered drink mixtures, buttermilk, and buttermilk powder.

The preferred mode of administration of the composition of the invention is by oral ingestion. However, it may be appropriate to administer the composition by other means, such as intravenously. The composition of the invention may be ingested, or otherwise administered, in any dosage levels and dosage frequencies suitable for controlling cholesterol, LDL and triglyceride levels. Typically, an amount of the composition will be ingested daily or weekly by a human.

It will also be clear to the skilled practitioner that the amount of β-casein A² to be ingested or otherwise administered will vary between subjects.

The invention is further described with reference to the following examples. However, it is to be appreciated that the invention is not limited to these examples.

### EXAMPLES

### Materials and Methods

Sixty New Zealand White/Lop cross rabbits of both sexes (16-24 weeks old) were obtained from the University of Queensland Central Animal Breeding House.

### Protocol summary

At time = 0, the right carotid artery of all rabbits was balloon de-endothelialised. The rabbits were then randomly divided into 10 Groups and fed the specified diets for 6 weeks. Blood samples were taken at t=0, 3 and 6 weeks for analysis of serum constituents. At t=6 weeks, all animals were sacrificed and tissue samples taken. A summary of the study protocol is shown in Table 1.

**Table 1: Protocol Summary**

| **Week** | **0** | **1** | **2** | **3** | **4** | **5** | **6** |
|---|---|---|---|---|---|---|---|
| **Surgery** | X | | | | | | |
| **Diet** | XXXXXXX | XXXXXXX | XXXXXXX | XXXXXXX | XXXXXXX | XXXXXXX | XXXXXXX |
| **Bleeds** | X | | | X | | | x |
| **Weights** | X | X | X | X | X | X | X |
| **Sacrifice** | | | | | | | X |

| | | | | | | | |
|---|---|---|---|---|---|---|---|
| Surgery at t=0; study pellets distributed daily; blood collection at t=0, 3, 6 weeks; weights recorded weekly; sacrifice at t=6 weeks | | | | | | | |

### Surgery

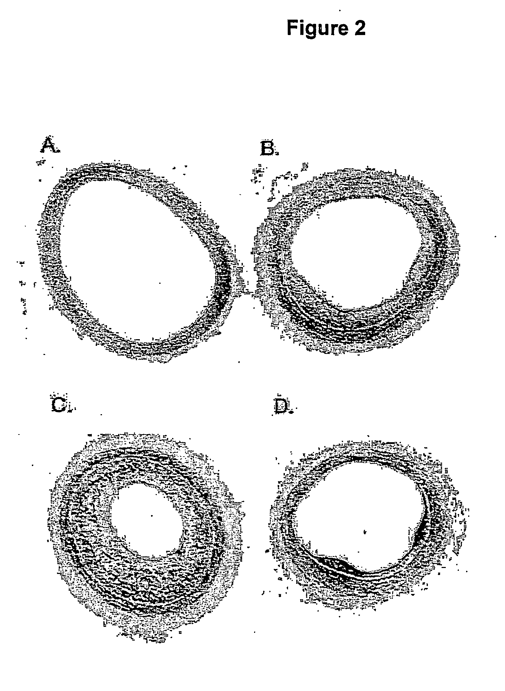

At time = 0, all 60 rabbits were anaesthetised with a mixture of ketamine (30 mg/kg) and xylazine (7 mg/kg) *i.m.* and maintained on Halothane (Laser Animal Health, Qld, Australia). An incision was made through skin and fascia, and blunt dissection and incision through the stylohyoid muscle exposed the upper part of the right common carotid artery. The left, uninjured common carotid artery served as a contralateral control. Side branches were temporarily ligated with 5/0 silk (Davis & Geck, Danbury, CT, USA) and a 2 French (2F) Fogarty embolectomy catheter (Baxter Healthcare Corporation, Irvine, CA, USA) was introduced through an arteriotomy made in the superior thyroid artery, a branch of the external carotid artery (Figure 2.2). The catheter was advanced 10 cm (to the aortic arch), the balloon inflated with 0.2 ml of saline and the catheter withdrawn in order to deendothelialise the artery.

The injury procedure was performed three times in each animal to ensure complete removal of the endothelium. After injury, the catheter was removed, the superior thyroid artery ligated with 5/0 silk and the wound closed using 2/0 Dexon braided sutures (Sherwood Medical, St. Louis, MO, USA). A broad-spectrum antibiotic (5 mg/kg Baytril, Bayer Australia Ltd, Pymble, Australia) containing 50 mg/ml enrofloxacin was administered sub-cutaneously at the completion of the procedure. The rabbits were monitored post operatively and returned to their cages when fully conscious. For three days post-surgery each animal was gavaged with approximately 170 mg of paracetamol (SmithKline Beecham International, Ermington, Australia) and sub-cutaneous administration of antibiotics was continued.

### Rabbit diets

The study involved 10 diets, the compositions of which are shown in Table 2. Essentially, each diet contained 20% total milk protein, but differed in the proportion of casein protein to whey protein. Diets of Groups 1, 9 and 10 contained no further additives, while diets of Groups 2, 3, 4, 5, 6, 7 and 8 were supplemented with 0.5% cholesterol. There were 6 rabbits in each group and the diet was continued for 6 weeks.

**Table 2 Compositions of study diets offered to rabbits for 6 weeks.**

| Diet Group | 1 | 2 | 3 | 4 | 5 | 6 | 7 | 8 | 9 | 10 |
|---|---|---|---|---|---|---|---|---|---|---|
| | Ingredients (grams / kg) | | | | | | | | | |
| β-casein A¹ | 0 | 0 | 50 | 100 | 200 | 0 | 0 | 0 | 100 | 0 |
| β-casein A² | 0 | 0 | 0 | 0 | 0 | 50 | 100 | 200 | 0 | 100 |
| Whey protein | 228 | 228 | 171 | 113.8 | 0 | 171 | 113.8 | 0 | 113.8 | 113.8 |
| Cholesterol | 0 | 5 | 5 | 5 | 5 | 5 | 5 | 5 | 0 | 0 |
| | | | | | | | | | | |
| DL -Methionine | 3 | 3 | 3 | 3 | 3 | 3 | 3 | 3 | 3 | 3 |
| Sucrose | 426.372 | 421.372 | 428.53 | 435.886 | 449.865 | 428.496 | 435.818 | 449.865 | 440.886 | 440.886 |
| Corn Oil | 10 | 10 | 10 | 10 | 10 | 10 | 10 | 10 | 10 | 10 |
| Coconut Oil | 130 | 130 | 130 | 130 | 130 | 130 | 130 | 130 | 130 | 130 |
| Cellulose, Fibre | 150 | 150 | 150 | 150 | 150 | 150 | 150 | 150 | 150 | 150 |
| Salt Mix #200951 | 40 | 40 | 40 | 40 | 40 | 40 | 40 | 40 | 40 | 40 |
| Vitamin Mix #320005 | 10 | 10 | 10 | 10 | 10 | 10 | 10 | 10 | 10 | 10 |
| Choline Bitartrate | 2 | 2 | 2 | 2 | 2 | 2 | 2 | 2 | 2 | 2 |
| Zinc premix (5mg/g) | 0.628 | 0.628 | 0.47 | 0.314 | 0.135 | 0.504 | 0.382 | 0.135 | 0.314 | 0.314 |
| | | | | | | | | | | |
| Total | 1000 | 1000 | 1000 | 1000 | 1000 | 1000 | 1000 | 1000 | 1000 | 1000 |

It was found that it took several days for the rabbits to become accustomed to their new diets, which differed from their normal pellets in colour, smell, texture and weight. Pellets fed in the new diets were white, while normal pellets are brown. Thus, for five days prior to surgery, all rabbits were weaned onto their respective diets by mixing increasing amounts of the new pellets (25%, 50%, 75%, 100%) with normal rabbit pellets (to total 200 grams). At t=0, only new pellets (200 grams per day) were offered (exceptions, see below) for the following 6 weeks and water was available *ad libitum.* It is known that rabbits eat 90 - 130 grams of pellets per day. However, 200 grams was required to fill the hopper such that the animals could reach the food. A fresh 200 grams of pellets was provided daily and the amount of pellets left uneaten by each rabbit was determined by weighing each day.

Several measures were employed to encourage the rabbits to eat the new diet. Firstly, a small amount of aniseed oil was dropped onto the back of the food hoppers to stimulate the rabbits' appetites. Secondly, if a rabbit was still not eating its new diet or its weight loss was approaching 10% of original body weight, normal food was added to the diet at 25, 50 or 75% mixtures. When this occurred, the white diet pellets were carefully separated from the brown normal pellets and weighed each day to determine the actual amount of experimental diet consumed. Finally, if the weight loss continued, the rabbits were offered 200 grams of normal food per day until their body weight increased.

### Rabbit weights

All rabbits were weighed weekly, commencing the day of surgery, until the week of sacrifice.

### Blood collection

Blood was collected from each rabbit prior to surgery, at the commencement of diet and at t=3 and 6 weeks. Each rabbit was wrapped securely and the fur covering the central ear artery was shaved. A 24 gauge catheter (Becton Diskinson Pty Ltd, NSW, Australia) was inserted and 5 mL of blood was collected into sterile tubes. The blood was allowed to clot and was centrifuged at 4500 rpm for 10 minutes. The serum supernatant was extracted and analysed for total serum cholesterol, triglycerides, HDL, LDL and homocysteine.

### Rabbit sacrifice

Six weeks after injury, 1000 I.U. heparin (David Bull Laboratories, Mulgrave, Victoria) in 1 ml saline were injected into an ear vein of each rabbit. Five minutes later, the rabbits were euthanased with an overdose of pentobarbitone (325 mg/ml Lethabarb, (Virbac Australia Pty Ltd, Victoria)). An incision was made from the chin to pelvis and continued to the hindlimbs and forelimbs. The peritoneal cavity was then carefully incised to expose internal organs and the rib cage was cut away to expose the heart. The left ventricle was cannulated with a wide gauge needle attached to tubing which was in turn connected to a perfusion apparatus. A small section of the inferior vena cava was cleared and severed. The circulatory system was flushed with 1 litre of Dulbecco's Phosphate Buffered Saline (DPBS) before arterial perfusion fixation was performed with 1 litre of 4% formaldehyde (Asia Pacific Speciality Chemicals Ltd, NSW, Australia) in DPBS at 100 mm Hg.

### Tissue collection

The entire aorta, from the iliac bifurcation to the aortic arch, was exposed and removed. The right (injured) and the left (control) common carotid arteries were fully exposed and removed. All vessels were cleaned of fat and adherent connective tissue before being placed into tubes with 4% formaldehyde in DPBS.

### Tissue analysis

### En face Oil-Red-O staining for luminal lesions

The aortic arch was removed for fixation and histological sectioning. The remainder of the aorta (approximately 16 cm) was opened longitudinally and cut into four segments. Each segment was sewn (lumen side up) onto small pieces of clear plastic to ensure they remained flat. The segments were then stained *en face* in a saturated solution of the lipophilic dye Oil Red O to enable determination of luminal surface area covered by fatty streaks.

### Analysis of luminal surface area covered by fatty streaks

Images of each aortic segment were scanned using Desk Scan II (version 2.31 a, Hewlett-Packard, U.S.A.) and the total lesion area was measured using the ImageJ imaging software (version 1.23y, National Institute of Health, U.S.A.). This program compares the intensity of the lesions (stained bright red) against the rest of the arterial surface (white to pale pink). The results were expressed as the percentage of surface area covered by lesions.

### Aortic arch

Three segments of approximately 3-4 mm in length were cut from each aortic arch and placed in DPBS for two washes of 30 minutes each. The segments were then dehydrated in increasing concentrations of ethanol, i.e. 70% ethanol for 30 minutes, then 95% ethanol for 30 minutes and finally, 100% ethanol for three washes of 30 minutes each. They were then placed in the first of two infiltration resin solutions (Technovit 7100, Heraeus, Germany) and left on a rotator overnight. The segments were then transferred to the second resin solution and were again left on a rotator overnight. Each segment was then carefully placed upright into embedding moulds, held in place by a minimal amount of superglue at the base of the mould. The same resin mixture used for infiltration was mixed with a hardener and this was pipetted into the mould until all segments were completely covered. The plastic was left to polymerise at room temperature for two hours before being placed into a 37°C oven overnight. The plastic blocks were then gently removed from their moulds and mounted onto Histoblocks, using Technovit 3040 (Heraeus, Germany) as glue. Each block was securely fitted to the microtome (LKB Bromma, Germany) and trimmed by cutting approximately 60-70 sections of 10 µm each. These were discarded and the microtome was set to cut 5 µm sections. These sections were collected with forceps and stretched in distilled water held at room temperature. They were then collected onto glass slides and dried for approximately 15 minutes at 60°C. The sections were then stained for 10 minutes with Toluidine Blue before being washed four times with tap water and allowed to air dry.

### Right and left carotid arteries

Four and five segments, each approximately 3-4 mm in length, were cut from the left (control) and the right (ballooned) carotid arteries respectively. A different number of sections was used in the control and the experimental vessels, to avoid confusion between tissues during embedding and sectioning. The segments were dehydrated, embedded, sectioned and stained using the method previously described for the aortic arch segments.

### Intima to media ratios

All morphometric analyses of the intima to media ratios (I:M) of the ballooned and control carotid arteries and the aortic arch were perfomed using the Mocha Image Analysis system (version 1.1, Jandel Scientific, Ca., U.S.A.). Measurements were taken to determine the size (in pixels) of both the intima and media layers in all segments of each artery. Using an Excel (Microsoft Corporation, N.Y., U.S.A.) spreadsheet, the ratio of intima to media was calculated and recorded for each of the three aortic arch segments per rabbit, the four control carotid segments and the five ballooned carotid segments.

### Statistics

All statistical analyses were performed using the SigmaStat (Jandel Scientific, Ca., U.S.A.) statistical software package. Comparison of data from the morphometric analyses were carried out with One-Way Multiple ANOVAs, using the "Kruskal-Wallis" Analysis of Variance on Ranks test. Extreme outliers were identified using Tukey's Outlier Test. In all statistical analyses a P value of less than 0.05 was considered significant. The information has been presented graphically using Microsoft Excel (Microsoft Corporation, N.Y., U.S.A.). All data has been reported as mean ± standard error of mean.

### Results

### Rabbit Diets

Rabbits were weaned onto their respective new diets for a few days prior to t = 0, and some animals whose weight loss approached 10% had their diet supplemented with normal pellets for a few days. Thus it was essential to measure the amount of new pellets eaten by each animal per day by providing a known weight (200 grams) of fresh pellets each morning and weighing the uneaten portion the following morning immediately prior to offering 200 grams of fresh pellets. Where a mixture of new pellets (white) and normal pellets (brown) was offered, the two were separated prior to weighing.

The weight of the white pellets eaten by each rabbit varied between rabbits. On group averages, the weight of pellets consumed only varied by 12 grams per day over the six week experimental period and ranged from 23.2 grams to 35.3 grams per day (mean 30.48 ± 0.97 grams). There was no significant difference in the amount of diet pellets eaten between any of the Groups. The addition of 0.5% cholesterol to the diets of Groups 2 - 8 did not influence the amount eaten, nor did the type or amount of casein. Thus, while the amount of food consumed by the rabbits was relatively low (mean 30.48 grams white pellets per day, mean 10.22 grams brown pellets per day, total mean 40.72 grams per day) resulting in some weight loss, the amount of the new diet consumed in each Group was relatively constant and thus results between Groups can be compared.

### Rabbit Weights

Almost all rabbits lost weight over 6 weeks, with only Group 9 (10% A¹ without added cholesterol) recording an average weight gain of 2.15 ± 1.2%. Group 10 rabbits (10% A² without added cholesterol) showed only a small weight loss of -0.92 ± 0.97%. Group 1 rabbits on 20% whey only (no added cholesterol) lost 4.5 ± 6% of their original body weight over the 6 week study, while the remaining Groups 2 - 8 (all with added cholesterol) lost mean 7.57 ± 4.72% of their original body weight.

The greatest mean weight loss was by Group 6. However this was entirely due to one rabbit (K6) that had a large starting weight of 3.81 kg. This rabbit ate an average of 21.58 grams of pellets per day, approximately 9 grams per day less than the mean of all rabbits (30.50). K6 showed no sign of illness and at the time of sacrifice and all organs appeared normal with the exception of a very small liver of normal colour. The remaining five rabbits in this Group lost less than 5% of their original body weight. There was no apparent relationship between proportion or type of casein on weight change.

### Effect of Diet on Cholesterol

At week 6 cholesterol levels of almost all Groups were significantly different from Group 1 (20% whey only). Interestingly, the cholesterol level in Group 9 (A¹) was higher than in Group 1, but this was not significant. Also, the cholesterol level in Group 8 (20% A² with cholesterol) was approximately half of that produced from 20% whey with added cholesterol (Group 2), although this too was not significant. Group 9 serum cholesterol was significantly lower than all Groups that had dietary cholesterol (Groups 2 - 8). Group 10 (A²) serum cholesterol was significantly lower than in Group 1, and indeed was significantly lower than that in all groups, including Group 9.

The results indicate that addition to 0.5% cholesterol to the diet has caused an increase in serum cholesterol levels irrespective of the amount of whey in the diet and both the amounts and types of casein. Also, in the absence of dietary cholesterol, A² produced lower serum cholesterol levels than both A¹ and whey.

### Effect of Diet on Triglycerides

By week 6 triglyceride levels from Groups 5 (20% A¹) and 9 (10% A¹ without added cholesterol) had increased from week 0 and Groups 4 (10% A¹) and 10 (10% A² without added cholesterol) had shown no change. The serum triglyceride levels for all other Groups had fallen from those at week 0.

### Effect of Diet on LDL

In all groups with added dietary cholesterol, serum LDL levels were significantly higher than Group 1 (whey alone) or Groups 9 (A¹) and 10 (A²) with no dietary cholesterol. Group 9 (A' with no dietary cholesterol) was not significantly different from Group 1, but Group 10 (A² with no dietary cholesterol) was significantly lower. Also Group 10 LDL levels were significantly lower than those for Group 9. All Groups with added dietary cholesterol (Groups 2 - 8) were not significantly different from each other.

Thus, serum LDL levels followed the same pattern as serum cholesterol and indicate that addition of dietary cholesterol has increased serum LDL levels in all appropriate groups, while the A² casein variant in the absence of dietary cholesterol, produced lower serum LDL than either A¹ or whey alone.

### Effect of Diet on HDL

Serum HDL followed the same pattern as serum LDL with added dietary cholesterol (Groups 2 - 8) causing significantly elevated levels than those with no added cholesterol (Groups 1, 9 and 10). Group 9 was not significantly different from Group 1, but was significantly higher than Group 10, which in turn was significantly lower than all Groups except Group 1. β-Casein A² without dietary cholesterol produced a lowered serum HDL level than A¹.

### Effect of Diet on LDL to HDL ratio

The LDL to HDL ratios were determined by dividing the individual rabbit's serum LDL levels by the same rabbit's serum HDL levels. The mean LDL:HDL for each Group was then determined. It was found that the LDL:HDL of Group 10 (10% β-casein A² alone) was significantly lower than all other groups including whey alone and β-casein A¹ alone. The LDL to HDL ratios of all Groups with dietary cholesterol (Groups 2 - 8) were not significantly different from each other. As with the serum LDL and HDL levels, there was a trend for higher levels with higher doses of β-casein A¹ and lower levels with higher doses of β-casein A².

### Effect of Diet on Homocysteine

By week 6, serum homocysteine levels had changed only slightly from those at week 0 with no significant difference demonstrated between the experimental groups.

### Effect of Diet on Atherosclerosis

Group 1 rabbits fed 20% whey had 0% aortic surface area covered by plaque: All other Groups had some plaque, including those from Group 10, although this was not significantly higher than Group 1. Only Groups 1 and 10 were significantly lower than Group 3, which was also considerably higher than Groups 2 - 9, however, variation between rabbits within this Group was great, with a range between 2.8 and 18.4%. Three rabbits were excluded from these calculations as they were deemed extreme outliers.

Group 1 thoracic aorta had 0% of the surface area covered by fatty streaks. All other Groups had some lesions, including Groups 9 (A¹) and 10 (A²) with no dietary cholesterol. Groups 9 and 10 were not significantly different from each other. For both A¹ and A² with dietary cholesterol, there was a trend to lower percent lesions with increasing concentration of casein variant, but this was not significant. Group 3 was considerably higher than all others, but not significantly different. Of the six rabbits in this Group, three had a high percentage of surface area covered by plaque and three had low values.

The abdominal aorta isolated from animals fed A² in the absence of dietary cholesterol (Group 10) had only minimal surface area covered by plaque and was not significantly different from Group 1 (whey only) which had 0% covered by plaque. Group 10 was significantly lower than all other Groups including Group 9 (A¹, no dietary cholesterol). Group 3 (5% A¹ with dietary cholesterol) was significantly higher than Group 2 with 20% whey and dietary cholesterol.

Thus, both casein variants without dietary cholesterol produce more extensive fatty streaks than whey alone but A² is less atherogenic than A¹. In addition, A¹ is more atherogenic than A² in the presence of dietary cholesterol.

### Intima to Media ratios

With 20% whey alone (Group 1) there was no intimal thickening of the aortic arch. Similarly, in the presence of 10% A² with no dietary cholesterol (Group 10) or with dietary cholesterol and 5% or 20% A² (Groups 6 and 8), the mean intima to media ratio was 0. Group 7 (10% A² with dietary cholesterol) had a mean intima to media ratio of only 0.01, with four of the six rabbits having 0%. All A¹ groups (both with and without dietary cholesterol) had higher intima to media ratios than groups fed A² casein and had intima to media ratios not significantly different from Group 2 (20% whey with 0.5% cholesterol). Indeed, 10% A¹ in the absence of dietary cholesterol produced the same intima to media ratio as 20% whey with 0.5% whey with 0.5% dietary cholesterol (0.03).

Thus, in relation to the thickness of fatty streak lesions in the aortic arch, A¹ casein, even in the absence of dietary cholesterol, produces lesions similar to that produced by 0.5% cholesterol. In contrast A², both in the presence and absence of dietary cholesterol, appears to be highly atheroprotective.

Balloon catheter injury to the right carotid artery t = 0 induced a hyperplastic neointimal thickening in all rabbits by 6 week. Measurement of intima to media ratios showed there was a slight, but not significant increase in neointimal thickening in Group 2 rabbits (20% whey with 0.5% cholesterol) compared with Group 1 (20% whey alone). Likewise, the groups fed 5, 10 and 20% A¹ plus 0.5% cholesterol had thicker, but not significantly different neointimas than Group 1. Groups fed 5, 10 and 20% A² plus 0.5% cholesterol all had thinner neointimas than their A¹ counterparts, but again this was not significant. Similarly, in the absence of dietary cholesterol the intima to media ratio in A² fed animals (Group 10) was lower than in the A¹-fed Group 9.

There was no neointimal thickening observed in all of the control vessels.

Although the invention has been described by way of example, it should be appreciated that variations and modifications may be made without departing from the scope of the claims. Furthermore, where known equivalents exist to specific features, such equivalents are incorporated as if specifically referred in this specification.

### INDUSTRIAL APPLICABILITY

The consumption of β-casein A² is useful for reducing serum cholesterol levels, circulating triglyceride levels, LDL concentrations and thus LDL:HDL ratios, and serum levels of apolipoprotein B. The method of the invention is therefore useful for preventing or treating various diseases associated with mammalian serum cholesterol levels, LDL cholesterol levels and triglyceride levels. Such diseases include hypercholesterolemia, hyperlipidemia, and atherosclerosis. The invention also provides a dietary supplement containing β-casein A².

## Claims

1. β-casein for use in the treatment of hypercholesterolemia, hyperlipidemia and atherosclerosis by reducing the serum level in a mammal of any one or more of the following:
a) cholesterol;
b) low density lipoprotein (LDL) cholesterol relative to high density lipoprotein (HDL) cholesterol;
c) low density lipoprotein (LDL) cholesterol;
d) very low density lipoprotein (VLDL) cholesterol;
e) apolipoprotein B; and
f) triglycerides
where the β-casein is comprised of at least 95% β-casein A².

2. The use as claimed in claim 1 where the β-casein is comprised solely of β-casein A².

3. The use as claimed in claim 1 or claim 2 where the composition is a food or food product.

4. The use as claimed in claim 3 where the food or food product is milk or a product containing or processed from milk.

5. The use as claimed in any one of claims 1 to 4 where the composition is a dietary supplement.

6. The use as claimed in claim 5 where the dietary supplement is a nutriceutical which is ingested for optimising any one or more of the group consisting of performance during exercise, weight loss, weight gain, muscle building and muscle repair.

7. The use as claimed in any one of claims 1 to 6 where the composition is milk containing β-casein where the β-casein is comprised of at least 95% β-casein A².

8. The use as claimed in claim 7 where the β-casein is comprised solely of β-casein A².

9. The use as claimed in any one of claims 1 to 8 where the composition has been formed by the addition of an amount of β-casein A² sufficient to alter serum cholesterol levels and/or serum triglyceride levels in a human.

10. The use as claimed in any one of claims 1 to 9 where the β-casein A² is obtained from the milk of bovine cattle or from the milk of yaks, buffalo, sheep or goats.

## Patentansprüche

1. β-Casein zur Verwendung bei der Behandlung von Hypercholesterinämie, Hyperlipidämie und Atherosklerose durch Verringern des Serumspiegels bei einem Säuger von einem oder mehreren der folgenden:
a) Cholesterin;
b) Cholesterin in Lipoprotein mit niedriger Dichte (LDL) relativ zu Cholesterin in Lipoprotein mit hoher Dichte (HDL);
c) Cholesterin in Lipoprotein mit niedriger Dichte (LDL);
d) Cholesterin in Lipoprotein mit sehr niedriger Dichte (VLDL);
e) Apolipoprotein B; und
f) Triglyceriden,
wo das β-Casein aus mindestens 95% β-Casein A² besteht.

2. Verwendung nach Anspruch 1, wo das β-Casein allein aus β-Casein A² besteht.

3. Verwendung nach Anspruch 1 oder Anspruch 2, wo die Zusammensetzung ein Lebensmittel oder Lebensmittelprodukt ist.

4. Verwendung nach Anspruch 3, wo das Lebensmittel oder Lebensmittelprodukt Milch oder ein Produkt, enthaltend oder verarbeitet aus Milch, ist.

5. Verwendung nach einem der Ansprüche 1 bis 4, wo die Zusammensetzung ein Nahrungsmittelzusatz ist.

6. Verwendung nach Anspruch 5, wo der Nahrungsmittelzusatz ein Nährstoffpharmazeutikum ist, welches mit der Nahrung aufgenommen wird, um eines oder mehrere von der Gruppe, bestehend aus Leistung während Belastung, Gewichtsverlust, Gewichtszunahme, Muskelaufbau und Muskelwiederherstellung, zu optimieren.

7. Verwendung nach einem der Ansprüche 1 bis 6, wo die Zusammensetzung Milch, enthaltend β-Casein, ist, wo das β-Casein aus mindestens 95% β-Casein A² besteht.

8. Verwendung nach Anspruch 7, wo das β-Casein allein aus β-Casein A² besteht.

9. Verwendung nach einem der Ansprüche 1 bis 8, wo die Zusammensetzung durch die Zugabe einer Menge von β-Casein A², ausreichend, um Serumcholesterinspiegel und/oder Serumtriglyceridspiegel bei einem Menschen zu ändern, erzeugt worden ist.

10. Verwendung nach einem der Ansprüche 1 bis 9, wo das β-Casein A² aus der Milch von Rindvieh oder aus der Milch von Yaks, Büffeln, Schafen oder Ziegen erhalten wird.

## Revendications

1. β-caséine à utiliser dans le traitement de l'hypercholestérolémie, de l'hyperlipidémie et de l'athérosclérose en réduisant le taux sérique de l'un quelconque ou de plusieurs des éléments suivants chez un mammifère:
a) cholestérol;
b) cholestérol à lipoprotéines de basse densité (LDL) par rapport au cholestérol à lipoprotéines de haute densité (HDL);
c) cholestérol à lipoprotéines de basse densité (LDL);
d) cholestérol à lipoprotéines de très basse densité (VLDL);
e) apolipoprotéine B et
f) triglycérides
où la β-caséine est constituée d'au moins 95% de β-caséine A².

2. L'utilisation telle que revendiquée dans la revendication 1, où la β-caséine est constituée uniquement de β-caséine A².

3. L'utilisation telle que revendiquée dans la revendication 1 ou la revendication 2, où la composition est un aliment ou un produit alimentaire.

4. L'utilisation telle que revendiquée dans la revendication 3, où l'aliment ou le produit alimentaire est du lait ou un produit contenant du lait ou transformé à partir du lait.

5. L'utilisation telle que revendiquée dans l'une quelconque des revendications 1 à 4, où la composition est un supplément alimentaire.

6. L'utilisation telle que revendiquée dans la revendication 5, où le supplément alimentaire est un nutraceutique qui est ingéré afin d'optimiser l'un quelconque ou plusieurs des éléments parmi le groupe consistant en: performance pendant l'exercice, perte de poids, gain de poids, développement musculaire et réparation musculaire.

7. L'utilisation telle que revendiquée dans l'une quelconque des revendications 1 à 6, où la composition est du lait contenant de la β-caséine où la β-caséine est constituée d'au moins 95% de β-caséine A²_{.}

8. L'utilisation telle que revendiquée dans la revendication 7, où la β-caséine est constituée uniquement de β-caséine A².

9. L'utilisation telle que revendiquée dans l'une quelconque des revendications 1 à 8, où la composition a été formée par l'addition d'une quantité de β-caséine A² suffisante pour modifier les taux sériques de cholestérol et/ou les taux sériques de triglycérides chez un être humain.

10. L'utilisation telle que revendiquée dans l'une quelconque des revendications 1 à 9, où la β-caséine A² est obtenue du lait de bétail bovin ou du lait de yacks, de buffles, de brebis ou de chèvres.
